# EUROPEAN PATENT APPLICATION

(11) **EP 2 405 018 A1**
(43) Date of publication of application: **11.01.2012**
(21) Application number: 10748811.6
(22) Date of filing: 04.03.2010
(51) Int. Cl.: C12Q 1/68, C12N 15/09

(54) **METHOD FOR DETERMINING FATTY ACID SYNTHESIS PATHWAY OF MICROORGANISM, AND PCR PRIMER SET FOR USE IN THE METHOD**

(30) Priority: 04.03.2009 JP 2009050906
(71) Applicant: University of Miyazaki, Miyazaki-shi Miyazaki 889-2192 (JP); Nippon Suisan Kaisha, Ltd., Chiyoda-ku Tokyo 100-8686 (JP); Kyushu University, National University Corporation, Fukuoka-shi Fukuoka 812-8581 (JP)
(72) Inventor: NAGANO, Naoki, Miyazaki-shi Miyazaki 889-2192 (JP); HAYASHI, Masahiro, Miyazaki-shi Miyazaki 889-2192 (JP); SAKAGUCHI, Keishi, Fukuoka-shi Fukuoka 812-8581 (JP); ITO, Makoto, Fukuoka-shi Fukuoka 812-8581 (JP); TAOKA, Yosuke, Tokyo 100-8686 (JP); OKITA, Yuji, Tokyo 100-8686 (JP)
(74) Representative: TBK
(86) International application number: PCT/JP2010/053553
(87) International publication number: WO 2010/101218

(57) **Abstract**

Disclosed is a method for determining the fatty acid synthesis pathway of a microorganism. Specifically disclosed is a method for determining the fatty acid synthesis pathway of a microorganism, which is **characterized by** producing degenerate primers for a fatty acid synthesis-related enzyme gene and determining the presence or absence of the fatty acid synthesis-related gene in the genome gene extracted from the microorganism using the degenerate primers. The sequences for degenerate primers for C20-elongase gene, which is a fatty acid synthesis-related enzyme gene, are ATHGARTWYTKBRTITTYGTICA (SEQ ID NO:1) and TARTRISWRTACATIADIAMRTG (SEQ ID NO:2), and the sequences for degenerate primers for Δ4-desaturase gene are GGNCAYCAYCMITAYACNAA (SEQ ID NO:3) and TCDATYTGRTGIBWIARNCC (SEQ ID NO:4). The microorganism is one belonging to the class *Labyrinthulea.* Also specifically disclosed is a PCR primer set for use in the determination method. The method is useful as a method for screening a microorganism capable of synthesizing a fatty acid.

## Description

### Technical Field

The present invention relates to a method for determining the fatty acid synthetic pathway of a microorganism, and to a primer set for the detection thereof. Specifically, the present invention relates to a microorganism fatty acid synthetic pathway determining method that includes producing degenerate primers for a fatty acid synthesis-related enzyme gene, and determining the presence or absence of the fatty acid synthesis-related gene in a microorganism using the degenerate primers, and to a PCR primer set and a probe set for use in the method. The present invention is useful as a method for screening microorganisms that synthesize fatty acids.

### Background Art

Polyunsaturated ω3-fatty acid and ω6-fatty acid are important components in animal and human nutrition. Polyunsaturated long-chain ω3-fatty acids, including eicosapentaenoic acid (EPA) and docosahexaenoic acid (DHA), have various roles in many aspects of health, including brain development in children, eye functions, hormone and other signaling substance syntheses, and prevention of cardiovascular disturbances, cancers, and diabetes (see Non-Patent Literature 1), and thus represent an important component in human nutrition. Production of polyunsaturated long-chain fatty acids is thus needed in this regard.

Meanwhile, microorganisms categorized in the class Labyrinthulea are known to produce long-chain unsaturated fatty acids. Concerning the microorganisms of the family *Thraustochytriaceae,* there is a report of a process that makes use of microorganisms of the genus *Schizochytrium* to produce a long-chain highly unsaturated fatty acid-containing phospholipid (see Patent Literature 1). Microorganisms of the genus *Thraustochytrium* capable of producing DHA are also reported (see Patent Literature 2). The unsaturated fatty acids can enrich food and/or feed, and thus a simple, economical producing process of these unsaturated fatty acids is greatly needed, particularly in the eukaryotic system. For efficient production of unsaturated fatty acids using a microorganism, it is important to find a means that can be used to efficiently determine a microorganism that produces fatty acids.

One conventionally known method of examining the presence or absence of a known gene in a different organism is to perform a PCR with degenerate primers produced from known amino acid sequences. The reason that the amino acid sequences of proteins are used is that they are often more conserved (more common) than gene base sequences. For example, in a report concerning a synthetic pyrethroid-resistant *Aphis gossypii* Glover, known degenerate primers and newly designed specific primers are used in combination to perform a PCR, and the product is digested with restriction enzyme to determine a synthetic pyrethroid resistant clone. In other reports, use of degenerate primers to determine the effectiveness of a PC herbicide in plants that have resistance to sulfonylurea herbicides and imidazolinone herbicides (see Patent Literature 3), and use of polycondensation primers in a method of determining the presence or absence of a barley uzu gene (see Patent Literature 4) are disclosed.
There are also reports of determining methods that perform a PCR with such degenerate primers for microorganisms of the class Labyrinthulea. For example, a method that determines the presence or absence of a PUFA-PKS gene is disclosed (see Patent Literature 5). Two biosynthetic pathways of long-chain highly unsaturated fatty acid are known in Labyrinthulea microorganisms. In one pathway, EPA and DHA are produced by a series of desaturations and elongations of saturated fatty acids such as palmitic acid by the actions of desaturases and elongases. In the other pathway, long-chain highly unsaturated fatty acids are produced with polyketide synthase. The gene for the latter biosynthetic pathway is known as PUFA-PKS gene, and its presence or absence can be determined by the method disclosed in Patent Literature 5. However, this method cannot determine the presence or absence of the genes in the former biosynthetic pathway.
A method that determines the presence or absence of PUFA-PKS from the fatty acid composition of a cultured microorganism is disclosed as another method of determining a microorganism that has the PUFA-PKS system (see Patent Literature 6). In this method, a microorganism is cultured under aerobic conditions and oxygen-free/low-oxygen conditions, and five YES or NO questions are asked based on the comparison of the resulting fatty acid compositions. In this method, more answers with "YES" are regarded as the sign that the microorganism includes the PUFA-PKS gene. However, the method is very laborious, and does not produce proportional direct evidence, compared to the methods that directly detect genes by PCR. The conclusion drawn from the method thus merely tells the extent of the sign.
Further, the methods of Patent Literatures 5 and 6 only provide information concerning the PUFA-PKS pathway, and cannot determine the presence or absence of the other important pathway that produces EPA and DHA with elongase and desaturase. Accordingly, there is a need for a method that can conveniently and reliably detect and determine this pathway.

### Citation List

### Patent Literature

Patent Literature 1: JP-A-2007-143479
Patent Literature 2: JP-A-2005-102680
Patent Literature 3: JP-A-2002-281983
Patent Literature 4: JP-A-2004-215605
Patent Literature 5: JP-T-2007-532105 (the term "JP-T" as used herein means a published Japanese translation of a PCT patent application)
Patent Literature 6: JP-T-2005-510203

### Non-Patent Literature

Non-Patent Literature 1: Poulos, A Lipids 30:1-14, 1995; Horrocks, LA, and Yeo YK Pharmacol Res 40:211-225, 1999

### Description of the Invention

### Problems that the Invention is to Solve

It is an object of the present invention to provide a method for determining the presence or absence of a fatty acid synthetic pathway in a microorganism, and a PCR primer set and a probe set for use in the method. More specifically, an object of the present invention is to provide a method for determining the presence or absence of a fatty acid synthetic pathway in a microorganism, usable for efficient screening of microorganisms that synthesize fatty acids, and a PCR primer set and a probe set for use in the method.

### Means for Solving the Problems

Under these circumstances, the present inventors conducted intensive studies to provide an efficient method for the screening of microorganisms that have a fatty acid synthetic pathway, and found an easy and fast distinction method, and a PCR primer set for use in the method. The present invention has been completed based on this finding.

The gist of the present invention lies in the technical matter recited in (1) to (5) below.
(1) A method for determining a fatty acid synthetic pathway of a microorganism,comprising producing degenerate primers for a fatty acid synthesis-related enzyme gene, and determining the presence or absence of the fatty acid synthesis-related gene in genomic DNA extracted from the microorganism using the degenerate primers.
(2) The method according to (1), wherein the fatty acid synthesis-related enzyme is a C20 elongase and/or a Δ4 desaturase.
(3) The method according to (1) or (2), wherein the sequences of the degenerate primers for a C20-elongase gene are sequence 1 (ATHGARTWYTKBRTITTYGTICA) and sequence 2 (TARTRISWRTACATIADIAMRTG).
(4) The method according to (1) or (2), wherein the sequences of the degenerate primers for a Δ4-desaturase gene are sequence 3 (GGNCAYCAYCMITAYACNAA) and sequence 4 (TCDATYTGRTGIBWIARNCC).
(5) The method according to any one of (1) to (4), wherein the microorganism belongs to the class Labyrinthulea.
(6) A primer set for detecting a fatty acid synthetic pathway of a microorganism,
   the primer set including:
   a primer having the base sequence of SEQ ID NO: 1 of the Sequence Listing, and a primer having the base sequence of SEQ ID NO: 2 of the Sequence Listing, and/or
   a primer having the base sequence of SEQ ID NO: 3 of the Sequence Listing, and a primer having the base sequence of SEQ ID NO: 4 of the Sequence Listing.
(7) A microorganism fatty acid synthetic pathway detection kit including the primers of the primer set of (6) as probes.

### Advantage of the Invention

The present invention provides a method useful for the screening of microorganisms that synthesize fatty acids. The invention also provides a PCR primer set and a probe set for use in the method.

### Brief Description of the Drawings

FIG. 1 represents the results of the alignment analysis of the amino acid sequence of C20 elongase in various microorganisms (Example 1); the underlines indicate the primer design sites; the amino acid sequences of the C20 elongase of the various microorganisms presented in FIG. 1 are represented by SEQ ID NOS: 5, 6, 7, 8, and 9 in order from the top to the bottom (in order from the bottom to the top of Table 1).
FIG. 2 represents the results of the alignment analysis of the amino acid sequence of Δ4 desaturase in various microorganisms (Example 1); the underlines indicate the primer design sites; the amino acid sequences of the Δ4 desaturase of the various microorganisms presented in FIG. 2 are represented by SEQ ID NOS: 10, 11, 12, 13, and 14 in order from the top to the bottom (in order from the 2nd, 3rd, 1st, 5th, and 4th microorganisms in Table 2).
FIG. 3 represents the result of the electrophoresis performed for C20 elongase in Example 2.
FIG. 4 represents the result of the electrophoresis performed for Δ4 desaturase in Example 2.

### Mode for Carrying Out the Invention

### [Fatty Acid Synthesis-Related Enzyme]

The present invention is for determining the presence or absence of a fatty acid-related enzyme gene in a microorganism with the use of degenerate primers. The fatty acid-related enzyme is not particularly limited to the types of microorganism and fatty acid synthesis-related enzyme. The representative examples are enzymes such as C20 elongase and □4 desaturase. It is known that these enzymes exist in microorganisms that belong to the class Labyrinthulea. The desaturase is an enzyme that forms a double bond at a certain position from the carbonyl group of a fatty acid. The elongase is an enzyme responsible for the elongation reaction of fatty acid.

### [Fatty Acid Synthesis-Related Enzyme Gene]

The present invention relates to a method for determining the fatty acid synthetic pathway of a microorganism, which comprising producing degenerate primers for a fatty acid synthesis-related enzyme gene, and determining the presence or absence of the fatty acid synthesis-related gene using the degenerate primers. The fatty acid synthesis-related enzyme gene is not particularly limited, as long as it is a gene that encodes an enzyme associated with the synthesis. The particularly preferred fatty acid synthesis-related enzyme genes are C20-elongase gene and/or Δ4-desaturase gene.

### [Degenerate Primers]

Because the amino acid sequences of proteins are generally more conserved (more common) compared to gene base sequences, a method is known that determines the presence or absence of a known amino acid sequence by PCR, using degenerate primers produced from this amino acid sequence. Specifically, degenerate primers for the gene of a fatty acid synthesis-related enzyme are produced from the amino acid sequence of this enzyme, and the degenerate primers are used for PCR reaction with the genomic DNA extracted from a tested microorganism to confirm the presence or absence of the enzyme gene in the extracted genomic DNA. The presence or absence of a fatty acid synthetic pathway in the microorganism can then be determined from the result.

### [Production of Degenerate Primers]

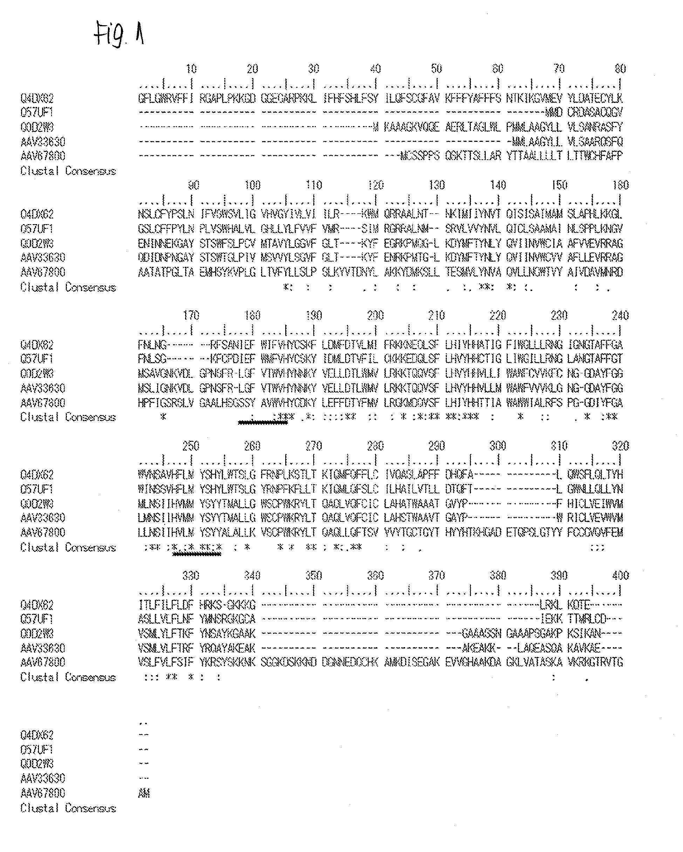

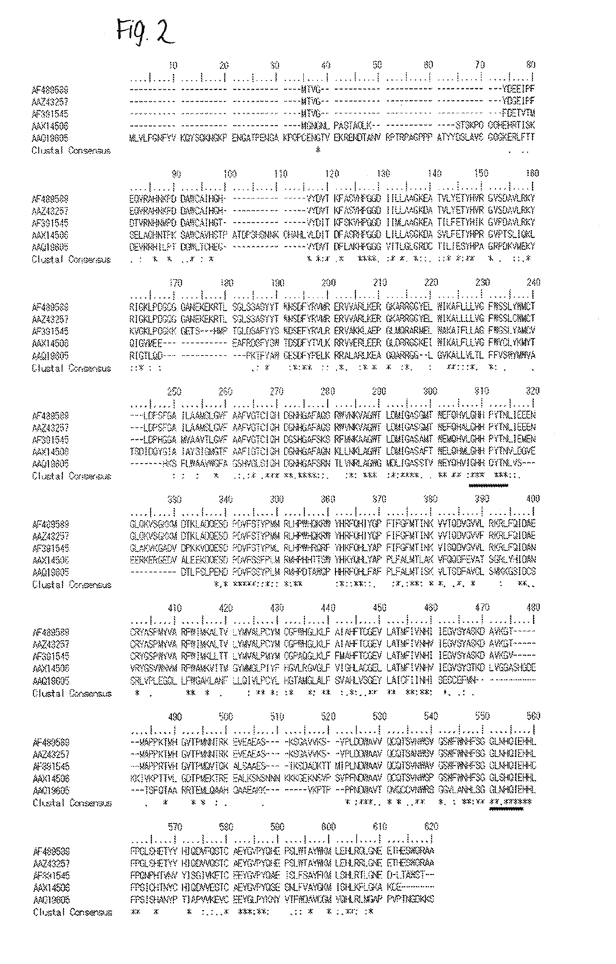

In the present invention, the degenerate primers for the fatty acid synthesis-related enzyme gene are produced as follows. For example, the amino acid sequences of the C20 elongase (Table 1, FIG. 1) and Δ4 desaturase (Table 2, FIG. 2) of various microorganisms available from Genbank are compared for homology by alignment analysis across the species. Degenerate primers of 20 to 23 base sequences are then designed based on the information of the amino acid sequences conserved across the species. Table 3 represents the degenerate primer sequences for different enzymes. In Table 3, the sequences are represented by SEQ ID NOS: 1, 2, 3, and 4 from the top. The degenerate primers may be designed from the sequences of the enzyme genes of various organisms. The degenerate primers correspond to the conserved regions of the amino acid sequence of the fatty acid synthesis-related enzyme.

### [Primer Set and Probe Set for Detection]

The presence or absence of a fatty acid synthetics pathway in a microorganism can be determined from the result of a test that detects the amplification of the enzyme gene by the PCR reaction of the designed primers with the genomic DNA extracted from the tested microorganism. PCR reaction may be performed by an ordinary method. For example, PCR may be performed under the following conditions: denaturation at 94°C for 60 seconds, followed by 30 cycles consisting of denaturation at 94°C for 30 seconds, annealing at 55°C for 30 seconds, extension and at 72°C for 30 to 60 seconds, and finally 7 minutes of extention at 72°C. The presence or absence of a fatty acid synthetic pathway in the microorganism can then be determined by confirming the amplification of the enzyme gene by methods such as electrophoresis.

In a preferred aspect of the present invention, the primer set for detecting a fatty acid synthetic pathway in a microorganism includes primers having the base sequences of SEQ ID NOS: 1 and 2, respectively, of the Sequence Listing, and/or primers having the base sequences of SEQ ID NOS: 3 and 4, respectively, of the Sequence Listing. Thus, in a preferred aspect, the probe set includes each primer of the primer set as a probe. A microorganism fatty acid synthetic pathway detection kit is realized by including these probes.

Note that the microorganism is not particularly limited, as long as it is thought to be capable of conducting fatty acid synthesis. The particularly preferred microorganisms are those belonging to the class Labyrinthulea. Examples of the class Labyrinthulea microorganisms include the genus *Labyrinthula, Althornia, Aplanochytrium, Japonochytrium, Labyrinthuloides, Schizochytrium, Thraustochytrium,* or the genus *Ulkenia* and *Aurantiochytrium.*

The following describes Examples of the present invention. Note, however, that the present invention is in no way limited by the following descriptions.

### Example 1

### Primer Design

The amino acid sequences of the C20 elongase (Table 1, FIG. 1) and Δ4 desaturase (Table 2, FIG. 2) of various microorganisms available from Genbank were compared for homology by an alignment analysis across the species. Degenerate primers of 20 to 23 base sequences were then designed based on the information of the amino acid sequences conserved across the species. Table 3 represents the degenerate primer sequences for each enzyme.

**[Table 1]**

| Species | GenBank Accession No. |
|---|---|
| *Thalassiosira pseudonana* | AAV67800 |
| *Pavlova* sp. | AAV33630 |
| *Pavlova salina* | Q0D2W3 |
| *Trypanosoma brucei* | Q57UF1 |
| *Trypanosoma cruzi* | Q4DX62 |

**[Table 2]**

| Species | GenBank Accession No. |
|---|---|
| *Thraustochytrium aureum* | AF391545 |
| *Thraustochytrium* sp. | AF489589 |
| *Thraustochytrium* sp. | AAZ43257 |
| *Euglena gracilis* | AAQ19605 |
| *Thalassiosira pseudonana* | AAX14506 |

**[Table 3]**

| Name | | Sequence | SEQ ID NO: |
|---|---|---|---|
| C20 elongase | | | |
| | EL020F | ATHGARTWYTKBRTITTYGTICA | 1 |
| | ELO20R | TARTRISWRTACATIADIAMRTG | 2 |
| | | | |
| Δ4 desaturase | | | |
| | DES4F | GGNCAYCAYCMITAYACNAA | 3 |
| | DES4R | TCDATYTGRTGIBWIARNCC | 4 |

| | | | |
|---|---|---|---|
| H=A/T/C, R=A/G, W=A/T, Y=C/T, K=G/T, B=C/G/T, S=C/G, M=A/C, N=A/C/G/T, I=inosine, D=A/T/G | | | |

### Example 2

### Determination of Fatty Acid Synthetic Pathway in Labyrinthulea

The degenerate primers obtained in Example 1 were used to determine the fatty acid synthetic pathways of six known species of Labyrinthulea. Table 4 presents the Labyrinthulea species tested. Note that these were all obtained from ATCC. Genomic DNA was extracted from these Labyrinthulea species, using an ISOPLANT (Nippon Gene) according to the protocol of the kit.

**[Table 4]**

| Species | ATCC number |
|---|---|
| *Thraustochytrium aureum* | ATCC 34304 |
| *Thraustochytrium roseum* | ATCC 28210 |
| *Thraustochytrium striatum* | ATCC 24473 |
| *Schizochytrium aggregatum* | ATCC 28209 |
| *Aurantiochytrium limacinum* SR21 | ATCC MYA-1381 |
| *Schizochytrium* (*Aurantiochytrium*) sp. | ATCC 20888 |

A PCR reaction was run for the genomic DNA extracted from each Labyrinthulea species, using the degenerate primers obtained in Example 1 for the fatty acid synthesis-related enzyme genes. The PCR conditions are presented in Table 5.

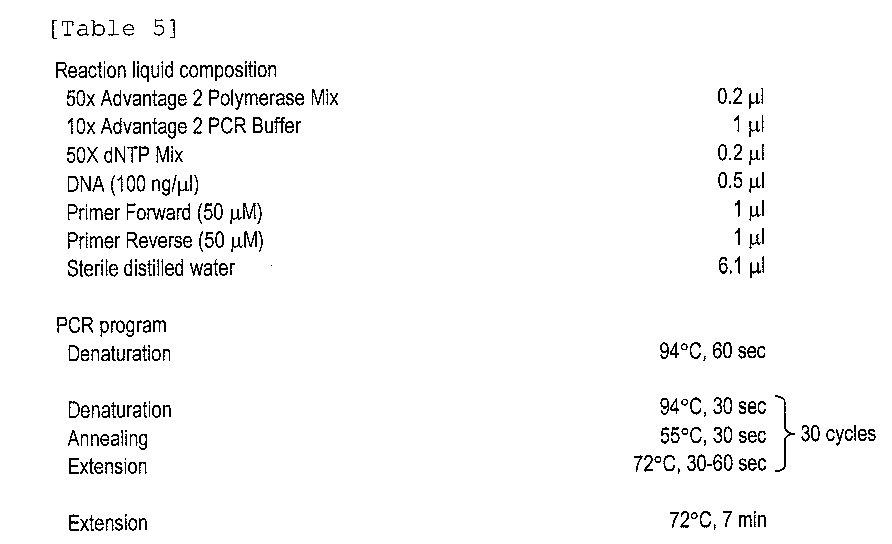

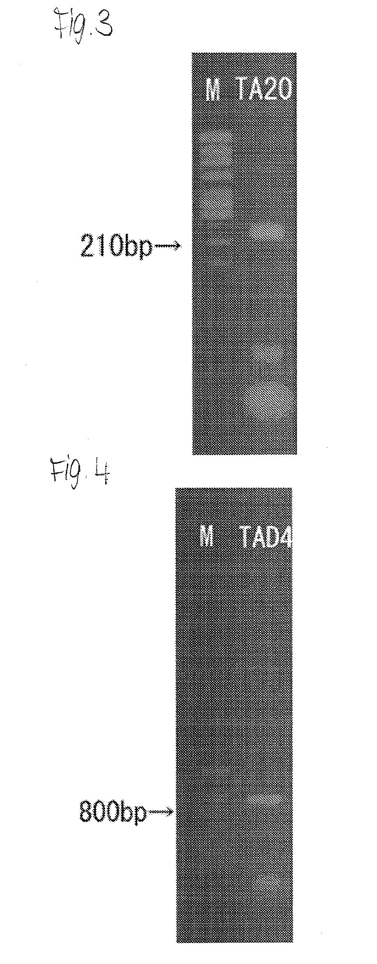

The result of the electrophoresis performed for the PCR product obtained by the PCR showed a band at about 230 bp for the C20 elongase (FIG. 3), and at about 700 bp for the Δ4 desaturase (FIG. 4). These bands were detected in *Thraustochytrium aureum* ATCC 34304, *Thraustochytrium roseum* ATCC 28210, *Thraustochytrium striatum* ATCC 24473, and *Schizochytrium aggregatum* ATCC 28209. However, neither band was detected in *Aurantiochytrium limacinum* SR21 ATCC MYA-1381 and *Schizochytrium* (*Aurantiochytrium*) sp. ATCC 20888.

Amplification of the target genes was confirmed. The target bands were cut from the electrophoresed gel, and purified using a Wizard SV gel and PCR clean-up system (Promega) according to the product manual. The PCR purified products were then inserted into a TA cloning vector after adjusting the concentration. Cloning was performed using a pGEM(R)-T Easy Vector System (Promega) according to the product manual. Extraction of the plasmid DNA was performed with a QIAprep Spin Miniprep Kit (QIAGEN). The extracted plasmid was subjected to sequence analysis. Sequence analysis was performed by a dye terminator method. The sequences of the resulting C20-elongase fragment and Δ4-desaturase fragment were then subjected to BLAST homology search (http://blast.ddbj.nig.ac.jp/top-j.html).

Table 6 presents the results of the homology search for the C20-elongase fragment. The C20-elongase fragments obtained from *Thraustochytrium aureum* ATCC 34304, *Thraustochytrium roseum* ATCC 28210, *Thraustochytrium striatum* ATCC 24473, and *Schizochytrium aggregatum* ATCC 28209 (the each sequence was represented as TAC20, TRC20, TSC20, and SAC20 ,respectively) had 40-48% amino acid sequence homology with the known C20-elongase gene available from database.

Table 7 presents the results of the homology search for the Δ4-desaturase fragment. The Δ4-desaturase fragments obtained from *Thraustochytrium aureum* ATCC 34304, *Thraustochytrium roseum* ATCC 28210, *Thraustochytrium striatum* ATCC 24473, and *Schizochytrium aggregatum* ATCC 28209 (the each sequence was represented as TAD4, TRD4, TSD4, and SAD4, respectively) had 78-97% amino acid sequence homology with the known Δ4-desaturase gene available from database.

As these results show, the C20-elongase and Δ4-desaturase genes were detected in the genus *Thraustochytrimu* and *Schizochytrium* with the degenerate primers designed for the detection of C20 elongase and Δ4 desaturase in the present invention. However, neither C20-elongase nor Δ4-desaturase gene was detected in the genus *Aurantiochytrium.* Specifically, the results are in conformity with the conventionally known attributes of Labyrinthulea, confirming that the present invention is useful as a method for determining the fatty acid synthetic pathway of Labyrinthulea.

**[Table 6]**

| Sequence name | Species | Species compared | Homology | GenBank Accession No. |
|---|---|---|---|---|
| TRC20 | *T. roseum* | *Pavlova* sp. CCMP459 | 40% | AAV33630 |
| SAC20 | *S. aggregatum* | *Pavlova viridis* | 48% | ABR67690 |
| TAC20 | *T. aureum* | *Pavlova* sp. CCMP459 | 40% | ABR67690 |
| TSC20 | *T. striatum* | *Pavlova* sp. CCMP459 | 40% | ABR67690 |

**[Table 7]**

| Sequence name | Species | Species compared | Homology | GenBank Accession No. |
|---|---|---|---|---|
| TRD4 | *T. roseum* | *Thraustochyirium aureum* | 97% | AAN75709 |
| SAD4 | *S. aggregatum* | *Thraustochyirium aureum* | 79% | AAN75709 |
| TAD4 | *T. aureum* | *Thraustochyirium aureum* | 96% | AAN75709 |
| TSD4 | *T. striatum* | *Thraustochyirium aureum* | 78% | AAN75709 |

### Industrial Applicability

The present invention concerns a method for determining the fatty acid synthesis pathway of a microorganism, whereby degenerate primers produced for a fatty acid synthesis-related enzyme gene are used to determine the presence or absence of the fatty acid synthesis-related gene in the genomic DNA extracted from the microorganism. The method enables the presence or absence of a fatty acid synthesis-related enzyme gene in a microorganism to be conveniently determined, and thus conveniently identifies a microorganism that produces unsaturated fatty acids usefully added to food and/or feed to enhance the health promoting effect. The present invention is particularly useful for the development and establishment of a technique for economically producing unsaturated fatty acids in the eukaryotic system. A progress in the unsaturated fatty acid producing technique makes it possible to provide substances useful to sustain healthy human life.

## Claims

1. A method for determining a fatty acid synthesis pathway of a microorganism,
comprising producing degenerate primers for a fatty acid synthesis-related enzyme gene, and determining the presence or absence of the fatty acid synthesis-related gene in genomic DNA extracted from the microorganism using the degenerate primers.

2. The method according to claim 1, wherein the fatty acid synthesis-related enzyme is a C20 elongase and/or a Δ4 desaturase.

3. The method according to claim 1 or 2, wherein the sequences of the degenerate primers for a C20-elongase gene comprise the base sequences of SEQ ID NOS: 1 and 2 of the Sequence Listing.

4. The method according to claim 1 or 2, wherein the sequences of the degenerate primers for a Δ4-desaturase gene comprise the base sequences of SEQ ID NOS: 3 and 4 of the Sequence Listing.

5. The method according to any one of claims 1 to 4, wherein the microorganism belongs to the class Labyrinthulea.

6. A primer set for detecting a fatty acid synthesis pathway of a microorganism,
comprising:
a primer having the base sequence of SEQ ID NO: 1 of the Sequence Listing, and a primer having the base sequence of SEQ ID NO: 2 of the Sequence Listing, and/or
a primer having the base sequence of SEQ ID NO: 3 of the Sequence Listing, and a primer having the base sequence of SEQ ID NO: 4 of the Sequence Listing.

7. A microorganism fatty acid synthesis pathway detection kit comprising the primers of the primer set of claim 6 as probes.
